# EUROPEAN PATENT APPLICATION

(11) **EP 1 716 846 A2**
(43) Date of publication of application: **02.11.2006**
(21) Application number: 06252226.3
(22) Date of filing: 26.04.2006
(51) Int. Cl.: A61K 9/08, A61K 47/18, A61K 47/30, A61K 47/38, A61K 47/46, A61K 31/00

(54) **Preparation and use of hydrogels**

(30) Priority: 26.04.2005 US 114820; 07.10.2005 US 245596
(71) Applicant: INTERNATIONAL FLAVORS & FRAGRANCES, INC., New York, NY 10019 (US)
(72) Inventor: Henson, Lulu, Plainsboro, New Jersey 08536 (US); Popplewell, Lewis Michael, Morganville, New Jersey 07751 (US); Qi-Zheng, June, Morganville, New Jersey 007751 (US); Toth, Adam J., Aberdeen, New Jersey 07747 (US); Bryant, Cory M., Washington DC 20010 (US); Hans, Keith, Franklin Park, New Jersey 08823 (US); Pringgosusanto, Franklin, Laurence Harbor, New Jersey 08879 (US)
(74) Representative: Lawrence, John

(57) **Abstract**

The present invention pertains, to a method for preparing a hydrogel in dry conditions that contains high loading of the active ingredient, preferably flavor or fragrance molecules. The hydrogel compositions are initially in solid form and preferably extruded and sized to form the desired shape and size. The actual hydrogel is formed in-situ from the uptake of water that is present in the suspending medium. The hydrogels have the following properties:
- contain of up to 40% of active ingredient prior to the water uptake;
- retain color and particle identity in high moisture systems; and
- retain color and particle identity at elevated temperature.

## Description

### Field of the Invention

The present invention relates to a method for preparing a hydrogel and use of the hydrogel in the flavor, fragrance, or pharmaceutical industries. In a preferred embodiment the hydrogel compositions are prepared by extrusion and contain flavor and fragrance products.

### Background of the Invention

Hydrogels are colloidal gels in which water is the dispersion medium. Hydrogel products are known in the art and are used in a wide variety of applications and products. Common uses for hydrogels are in disposable diapers, contact lenses, dressings for wounds, and breast implants.

US Patent 5,489,437 discloses an adhesive hydrogel product comprising a mixture containing water, and a thermoplastic water soluble polymer extruded in a dry state. The mixture is exposed to radiation to provide a breaking strength in excess of at least 10 pounds per square inch.

In a similar application, as disclosed in US 6,179,862, hydrogels are used to provide in-situ tissue adherent barriers as they are spayed onto tissue and then crosslinked. The crosslinkable solution is kept separate and are atomized and mixed in a gas stream prior to being applied to the tissue.

Another application of hydrogels is the delivery of therapeutic agents and diagnostic applications at high concentrations in condensate phase microparticles. US Patent No. 5,654,006 discloses that hydrogels having a particle size of from 0.5 to 5 microns are effective in the delivery of therapeutics, preferably therapeutics having a positive charge.

Despite the teachings of the above use of hydrogel materials, there is an ongoing need to develop new uses for hydrogel materials.

Nothing in any of the foregoing references discloses techniques for the production of the hydrogels of our invention or processes for producing same, with said hydrogels having advantageous properties with respect to the delayed release of active materials.

### Summary of the Invention

The present invention describes a method for preparation of a hydrogel comprising extruding the functional polymer and the active ingredient in dry conditions and forming a hydrogel in-situ by contacting the extruded material with water provided by the substrate. The unique feature of the invention is that prior to the composition contacting water in the substrate, no water is being added to this composition.

As an alternative, a hydrogel composition may be prepared by combining the functional polymer and the active ingredient in a spray dry emulsion followed by spray drying. The hydrogel is formed by contacting the dry product with water provided by the substrate.

A second embodiment of the invention is the use of cross-linking agents to impart thermal stability to the hydrogels, for example with hydrogels made with gelatin. This can be achieved either enzymatically, using transglutaminase or chemically using appropriate cross-linking agents.

In a third embodiment of the invention the composition of a hydrogel is prepared by admixing the ingredients of the invention together, and providing a heating source such as an extruder to obtain a melted matrix and then creating the desired particle size by forming, cooling, and sizing operations. The extruded material is added to a water-containing medium to form a hydrogel. The hydrogels are formed in-situ.

The formed hydrogels have the following properties:
- contain of up to 40% of active ingredient prior to the water uptake;
- retain color and particle identity in high moisture systems; and
- retain color and particle identity at elevated temperature.

These and other embodiments of the present invention will become apparent by reading the detailed description and accompanying examples.

A fourth embodiment object of the invention provides hydrogel particles that exhibit movement when incorporated into carbonated beverages.

### Detailed Description of the Invention

The retention of color, particle identity, and actives are provided to the hydrogels through the proper selection and combination of functional polymer, colorant, active ingredient, other functional additives, and processing conditions. Proper selection and combination of the above components of the hydrogel composition and processing conditions allow stable hydrogels to be formed. The hydrogel does not need to be irradiated with ionization energy for the cross-linking to be achieved. Suitable functional polymers include alginate, gelatin, gluten, starch, agar, xanthan gum, gellan gum, pectin, guar gum, hydroxypropyl methylcellulose (HPMC), methyl cellulose, microcrystalline cellulose, soy protein, whey protein, casein, collagen, hydrolyzed gelatin, and the like. The preferred materials are gelatin, gluten, instant starch, and sodium alginate. The level of functional polymer used in the preparation of the hydrogel, before the uptake of water is from about 1 to about 60 weight percent of the material to be extruded. Preferably the amount of the functional polymer ranges from about 7 to about 50 weight percent. The polymer used has a molecular weight of less then 250,000. Preferably, the polymer used has a molecular weight of less than 40,000.

The active ingredient provided by the present invention is preferably a hydrophobic material. The hydrophobic material is incorporated into a hydrogel composition thereby allowing it to be delivered in hydrophilic systems. The active ingredient may be any suitable material including therapeutic and diagnostic agents, flavors, fragrances and the like.

A list of suitable fragrances is provided in US Pat. No. 4,534,891. Another source of suitable fragrances is found in Perfumes, Cosmetics and Soaps, Second Edition, edited by W. A. Poucher, 1959. Among the fragrances provided in this treatise are acacia, cassie, chypre, cyclamen, fern, gardenia, hawthorn, heliotrope, honeysuckle, hyacinth, jasmine, lilac, lily, magnolia, mimosa, narcissus, freshly-cut hay, orange blossom, orchid, reseda, sweet pea, trefle, tuberose, vanilla, violet, wallflower, and the like.

Conventional flavoring materials useful in flavoring products include saturated fatty acids, unsaturated fatty acids and amino acids; alcohols including primary and secondary alcohols, esters, carbonyl compounds including ketones, other than the dienalkylamides of our invention and aldehydes; lactones; other cyclic organic materials including benzene derivatives, acyclic compounds, heterocyclics such as furans, pyridines, pyrazines and the like; sulfur-containing compounds including thiols, sulfides, disulfides and the like; proteins; lipids, carbohydrates; so-called flavor potentiators such as monosodium glutamate; magnesium glutamate, calcium glutamate, guanylates and inosinates; natural flavoring materials such as hydrolyzates, cocoa, vanilla and caramel; essential oils and extracts such as anise oil, clove oil and the like and artificial flavoring materials such as vanillin, ethyl vanillin and the like.

Specific flavor adjuvants include but are not limited to the following: anise oil; ethyl-2-methyl butyrate; vanillin; cis-3-heptenol; cis-3-hexenol; trans-2-heptenal; butyl valerate; 2,3-diethyl pyrazine; methyl cyclo-pentenolone; benzaldehyde; valerian oil; 3,4-dimethoxy-phenol; amyl acetate; amyl cinnamate; γ-butyryl lactone; furfural; trimethyl pyrazine; phenyl acetic acid; isovaleraldehyde; ethyl maltol; ethyl vanillin; ethyl valerate; ethyl butyrate; cocoa extract; coffee extract; peppermint oil; spearmint oil; clove oil; anethol; cardamom oil; wintergreen oil; cinnamic aldehyde; ethyl-2-methyl valerate; γ-hexenyl lactone; 2,4-decadienal; 2,4-heptadienal; methyl thiazole alcohol (4-methyl-5-β-hydroxyethyl thiazole); 2-methyl butanethiol; 4-mercapto-2-butanone; 3-mercapto-2-pentanone; 1-mercapto-2-propane; benzaldehyde; furfural; furfuryl alcohol; 2-mercapto propionic acid; alkyl pyrazine; methyl pyrazine; 2-ethyl-3-methyl pyrazine; tetramethyl pyrazine; polysulfides; dipropyl disulfide; methyl benzyl disulfide; alkyl thiophene; 2,3-dimethyl thiophene; 5-methyl furfural; acetyl furan; 2,4-decadienal; guiacol; phenyl acetaldehyde; β-decalactone; d-limonene; acetoin; amyl acetate; maltol; ethyl butyrate; levulinic acid; piperonal; ethyl acetate; n-octanal; n-pentanal; n-hexanal; diacetyl; monosodium glutamate; monopotassium glutamate; sulfur-containing amino acids, e.g., cysteine; hydrolyzed vegetable protein; 2-methylfuran-3-thiol; 2-methyldihydrofuran-3-thiol; 2,5-dimethylfuran-3-thiol; hydrolyzed fish protein; tetramethyl pyrazine; propylpropenyl disulfide; propylpropenyl trisulfide; diallyl disulfide; diallyl trisulfide; dipropenyl disulfide; dipropenyl trisulfide; 4-methyl-2-[(methyl-thio)-ethyl]-1,3-dithiolane; 4,5-dimethyl-2-(methylthiomethyl)-1,3-dithiolne; and 4-methyl-2-(methylthiomethyl)-1,3-dithiolane. These and other flavor ingredients are provided in U.S. Patent Nos. 6,110,520 and 6,333,180.

Examples of the appropriate therapeutic agents include hypnotics, sedatives, antiepileptics, awakening agents, psychoneurotropic agents, neuromuscular blocking agents, antispasmodic agents, antihistaminics, antiallergics, cardiotonics, antiarrhythmics, diuretics, hypotensives, vasopressors, antitussive expectorants, thyroid hormones, sexual hormones, antidiabetics, antitumor agents, antibiotics and chemotherapeutics, and narcotics.

Examples of diagnostic agents include, but are not limited to synthetic inorganic and organic compounds, proteins, peptides, polypeptides, polysaccharides and other sugars, lipids, and DNA and RNA nucleic acid sequences having diagnostic activities.

The level of active ingredient used in the preparation of the hydrogel, before the uptake of water is from about 5 to about 40 weight percent. Preferably the amount of the active ingredient ranges from about 10 to about 20 weight percent. High levels, such as 20 weight percent or greater, of the active ingredient can be achieved by the addition of appropriate functional materials such as modified starch, hydroxypropylcellulose, and/or ethylcellulose.

The functional additives in the hydrogel compositions are determined by the functional polymer in the composition. For example, a hydrogel composition with sodium alginate as the functional polymer will preferably employ a calcium salt as the functional additive. Calcium functions as a cross-linking agent for the alginate. The invention is advantageous in that the dry extruded composition combines the alginate and calcium in a unique polymer network capable of forming a hydrogel in-situ, thereby eliminating the need to solubilize the alginate and calcium separately prior to forming the hydrogel. The calcium salt may possess certain properties, such as slowly soluble in water and possess desirable melting temperature. The preferred calcium salt is calcium gluconate.

Certain naturally-occurring enzymes are good cross-linking agents. Such enzymes work by catalyzing the formation of bonds between certain amino acid side chains in proteins. Another hydrogel composition based on the use of either gelatin or gluten or other protein as the functional polymer may be used with a transglutaminase enzyme as the functional additive (Activa® Ajinomoto USA, Inc., Chicago, IL). This reaction imparts thermal stability to the hydrogel. Transglutaminase catalyzes an acyl transfer reaction between y-carboxamide groups of glutamine residues in a peptide and various primary amines, frequently e-amino groups of peptide-bound lysine residues. The result is a bond or cross-linkage between a glutamine residue in one protein molecule and a lysine residue in another protein molecule. It also minimizes color leaching from particles containing water miscible colorants such as lakes. Optimum conditions are required to achieve cross-linking within a reasonable amount of time, such as moisture, temperature of about 50ºC, pH 6-7, and high enzyme concentration, as described in JP 5292899. US Patent No. 6,325,951 discloses that it is possible to achieve cross-linking at lower temperatures, such as 5-10ºC, over a 16-hour period or at 5-40ºC for a period of from 10 minutes to 24 hours, see US Patent No. 5,658,605. In both cases, the level of available moisture is very high. These conditions can be modified depending on the raw materials. Surprisingly, it has been found that cross-linking can be achieved during extrusion, in dry conditions, with the water content of the mixture less then 5%, preferably less than 1%, most preferably no intentionally added water, within the residence time of the materials in the extruder. A powder form of the enzyme is conveniently mixed with the other dry ingredients of the extrusion powder blend. Extrusion is carried out within the time and temperature conditions typically used for melt extrusion.

It has also been found that enzymatic cross-linking can be achieved using typical conditions for preparing a spray dry emulsion and subsequent spray drying operation. The enzyme preparation is mixed with the dry ingredients and mixed with water to fully hydrate the ingredients. The flavor is added to the mixture and shear is applied to form a pre-emulsion followed by the spray drying operation.

An alternative functional additive for proteinaceous polymers is glutaraldehyde, formaldehyde or other aldehyde cross-linking materials. The level of functional additive used in the preparation of the hydrogel, before the uptake of water is from about 0.1 to about 20 weight percent of the material to be extruded. Preferably the amount of the functional additive ranges from about 0.5 to about 10 weight percent.

The colorants of the present invention include, but are not limited to lakes, preparations containing lakes, oleoresins, pigments, and minerals. An example of a preparation containing lakes is Spectra Flecks™ (Sensient Technologies, St. Louis, MO). Preferred colorants are botanical materials. Use of botanical materials reduces color leaching. Examples of botanical materials include ground tea leaves, dried parsley, dried red bell pepper, fruit powders, annatto, turmeric, beta-carotene, among others. Examples of pigments are cosmetic colorants identified by their INCI name CI 74260, CI 74160, CI 73360, among others. An example of a mineral is titanium dioxide. The level of colorant used in the preparation of the hydrogel, before the uptake of water is from about 0.01 to about 10 weight percent of the material to be extruded. Preferably the amount of the colorant ranges from about 0.1 to about 5 weight percent.

In addition to the foregoing components, various optional ingredients such as are conventionally used in the art, may be employed in the matrix of this invention. For example, fillers, diluents, emulsifiers, preservatives, anti-oxidants, stabilizers, lubricants, and the like may be employed herein if desired. Fillers include, for example, materials such as silicon dioxide, titanium dioxide, alumina, talc, kaolin, powdered cellulose and microcrystalline cellulose, as well as soluble materials such as mannitol, urea, sucrose, lactose, dextrose, sodium chloride and sorbitol. Diluents are typically necessary to increase bulk so that a practical size hydrogel is ultimately provided. Suitable diluents include calcium phosphate, calcium sulfate, carboxymethylcellulose calcium, cellulose, cellulose acetate, dextrates, dextrin, dextrose, fructose, glyceryl palmitostearate, hydrogenated vegetable oil, kaolin, lactitol, lactose, magnesium carbonate, magnesium oxide, maltitol, maltodextrin, maltose, microcrystalline cellulose, polymethacrylates, powdered cellulose, pregelatinized starch, silicified microcrystalline cellulose, sodium chloride, sorbitol, starch, sucrose, sugar, talc, hydrogenated vegetable oil, and mixtures thereof. Emulsifiers include mono and diglycerol esters of fatty acids, modified starch, polyglycerol esters, and sorbitol esters, preferably, the emulsifier is lecithin.

The formed hydrogels have the following properties:
- contain of up to 40% of active ingredient prior to the water uptake;
- retain color and particle identity in high moisture systems; and
- retain color and particle identity at elevated temperature.
Retention of color is understood to mean that no significant color leaching from the hydrogel occurs. Retention of particle identity means the hydrogel particles maintain their shape and appearance after being exposed to elevated temperatures or stress. The hydrogel composition of the invention retains color when heated to elevated temperatures of from about 75ºC to about 120ºC. Further, the hydrogel particles retain color and particle integrity when incorporated into a high moisture medium, at least 80% water, and heated to at least 75°C for at least 30 minutes.

In another embodiment of the invention, the hydrogel is used for delivering an active ingredient, such as flavor, into an ingestible composition. In this embodiment, the hydrogel is prepared by any of the methods described above and added to an ingestible composition, such as yogurt.

In a further embodiment of the invention it has been unexpectedly found that when the hydrogel particles of the present invention are incorporated into carbonated beverages they exhibit movement within the beverage due to the release of carbonation.

Certain factors may be taken into consideration to provide the desired effect such as the particle density (both initially upon introduction into the beverage and during the desired life), the particle size, the particle shape and ability to provide nucleation sites for bubble development, the beverage density, viscosity, the amount of carbonation, the temperature of carbonation release, the size and shape of beverage container and other physical characteristics.

It is also desirable that the particles remain essentially intact over the life of the movement desired, as well as any storage time required prior to carbonation release. Thus, particles that are essentially insoluble in the beverage matrix, as well as those that have appropriate solubility rates may be useful. The appropriate solubility rates may be achieved by the selection of appropriate polymers, additives and active ingredients. Normally, an essentially insoluble particle is desired in order to maintain particle movement for an acceptable time.

The particles may be added to a beverage immediately before consumption (e.g. added to a glass of beer just prior to pouring from a bottle/can or dispensing from a tap). They may also be formulated into a dry mix which is to be reconstituted with or added to a carbonated beverage, or is to be carbonated after addition (e.g. added to a powdered soft drink which is to be reconstituted with seltzer). The dry beverage mix typically contains at least one flavoring agent and/or taste-enhancing ingredient, sugar, acid, color, flavor and other diluents. In a further embodiment, the particles may be added to a beverage either pre or post-carbonation (e.g. added to bottles or cans of beer during packaging).

The particles may contain active materials that may impart a benefit to the beverage. Active materials may be selected from caffeine, extracts, nutritional supplements and flavors. Also the particles may further comprise colorants.

The particles have a size permitting a consumer to visually observe the resulting independent movement of them through the carbonated beverage, creating an interesting and entertaining visual effect. In one embodiment, the composite particles have a visually discernible size that is about 1mm to 5mm and more preferably about 4mm. In general, the composite particles have a size ranging between about 0.5mm to about 4mm, although they are not limited thereto. Composite particle sizes are generally limited on the high side by practical considerations of maintaining a size that can be suspended in a liquid without settling problems and which can be observed.

These particles are useful for a variety of carbonated beverages such as, but not limited, to beer and malt beverages, champagnes and sparkling wines, formulated alcoholic beverages such as wine spritzers, unflavored and flavored seltzer, and regular and reduced calorie sodas.

In all methods of preparation of a hydrogel as described above, spray drying may be used as an alternative to extrusion.

In order to demonstrate the invention, the following examples were conducted. All U.S. Patent and Patent applications referenced herein are hereby incorporated by reference as if set forth in their entirety. The following disclosures are provided to exemplify the present invention.

Unless noted to the contrary all weights are weight percent. Upon review of the foregoing, numerous adaptations, modifications and alterations will occur to the reviewer. These adaptations, modifications, and alterations will all be within the spirit of the invention. Accordingly, reference should be made to the appended claims in order to ascertain the scope of the present invention.

### EXAMPLE 1:

The following formulation was processed via extrusion:

| | % by weight |
|---|---|
| Strawberry Flavor | 10 |
| Silicon Dioxide | 5 |
| Emulsifier | 2 |
| Sorbitol | 30.8 |
| Gelatin | 50 |
| Transglutaminase | 2 |
| Color lake red #40 | 0.2 |

| | |
|---|---|
| (Extruder zone temperatures ºC: 1 = 40-50, 2 = 50-60, 3 = 55-65, 4 = 60-75, 5 = 90-105. Extruded melt was cooled, milled, and sized to give a specific particle size.) | |

### EXAMPLE 2:

Product from Example 1 was incorporated into a lemon-flavored chewy candy base at 100ºC. The particles survived the initial mixing and pulling process, retaining their visual identity and color in the candy. When the chewy candy was evaluated, it was observed that the strawberry flavor from the particles was not detected until the particles were masticated. Flavor from the particles continued to increase in intensity as chewing progressed. The example demonstrates that the flavor was protected in the hydrogel at a temperature of 100ºC.

### EXAMPLE 3:

Product from Example 1 was incorporated into a vanilla-flavored yogurt and stored in the refrigerator. The particles survived the mixing process, retaining their visual identity and color in the yogurt. When the yogurt was evaluated after 2 weeks, it was observed that there was a slight leaching of the color and flavor in the base. The particles remained intact and the color distinctly red. The particles retained a high level of strawberry flavor. No further changes were noted after 4 weeks of storage. This example demonstrates the stability of a gelatin-based hydrogel in a high moisture food product.

### EXAMPLE 4:

The following formulation was processed via extrusion:

| | % by weight |
|---|---|
| Orange Flavor | 10 |
| Silicon Dioxide | 5 |
| Emulsifier | 2 |
| Sorbitol | 30.8 |
| Gluten | 50 |
| Transglutaminase | 2 |
| Color lake yellow #5 & #6 | 0.2 |

| | |
|---|---|
| (The process followed was similar to that provided in Example 1.) | |

### EXAMPLE 5:

Product from Example 4 was incorporated into a vanilla-flavored yogurt and stored in the refrigerator. The particles survived the mixing process, retaining their visual identity and color in the yogurt. When the yogurt was evaluated after 2 weeks, it was observed that there was a slight leaching of the color and flavor in the yogurt. The particles remained intact and the color distinctly orange. The particles retained a high level of orange flavor. No further changes were noted after 4 weeks of storage. This example demonstrates the stability of a gluten-based hydrogel.

### EXAMPLE 6:

The following formulation was processed via extrusion:

| | % by weight |
|---|---|
| Peppermint Flavor | 20 |
| Silicon Dioxide | 6 |
| Emulsifier | 4 |
| Sorbitol | 17.99 |
| Gelatin | 50 |
| Transglutaminase | 2 |
| Color CI #74160 | 0.01 |

| | |
|---|---|
| (The process followed was similar to that provided in Example 1.) | |

### EXAMPLE 7:

Product from Example 6 was incorporated into a model toothpaste base. The particles survived the initial mixing process, retaining their visual identity and color in the toothpaste. This example demonstrates the stability of the hydrogel.

### EXAMPLE 8:

Product from Example 6 was incorporated into a chewy candy base at 100ºC. The particles survived the initial mixing and pulling process, retaining their visual identity and color in the candy. This example demonstrates the stability of the hydrogel.

### EXAMPLE 9:

The following formulation was processed via extrusion:

| | % by weight |
|---|---|
| Strawberry Flavor | 5 |
| Silicon Dioxide | 5 |
| Emulsifier | 2 |
| Maltitol | 37 |
| Starch Advantagel P75 | 50 |
| Color Red Spectra Flecks™ | 1 |

| | |
|---|---|
| (The process followed was similar to that provided in Example 1.) | |

### EXAMPLE 10:

Product from Example 9 was incorporated into a vanilla-flavored yogurt and stored in the refrigerator. The particles survived the mixing process, retaining their visual identity and color in the yogurt. When the yogurt was evaluated after 2 weeks, it was observed that there was a slight leaching of the color and flavor in the yogurt. The particles remained intact and the color distinctly red. The particles retained a high level of strawberry flavor. At 4 weeks, the particles were softer in texture and color intensity had decreased. This example demonstrates the stability of a starch-based hydrogel.

### EXAMPLE 11:

The following formulation was processed via extrusion:

| | % by weight |
|---|---|
| Peppermint Flavor | 5 |
| Silicon Dioxide | 5 |
| Emulsifier | 2 |
| Maltitol | 75.5 |
| Sodium Alginate | 7.5 |
| Parsley, milled | 5 |

| | |
|---|---|
| (The process followed was similar to that provided in Example 1.) | |

### EXAMPLE 12:

Product from Example 11 was incorporated into a vanilla-flavored yogurt and stored in the refrigerator. The particles survived the mixing process, retaining their visual identity and color in the yogurt. When the yogurt was evaluated after 2 weeks, it was observed that there was a slight leaching of the flavor in the yogurt. No parsley flavor was detected in the yogurt. No color leaching was observed. At 4 weeks, the particles were softer in texture and color intensity had decreased. This example demonstrates the color stability of an alginate-based hydrogel using a botanical material as the colorant.

### EXAMPLE 13:

The following formulation was processed via extrusion:

| | % by weight |
|---|---|
| Strawberry Flavor | 5 |
| Silicon Dioxide | 5 |
| Emulsifier | 2 |
| Maltitol | 75.3 |
| Sodium Alginate | 7.5 |
| Calcium Gluconate | 5 |
| Color Red Spectra Flecks™ | 0.2 |

| | |
|---|---|
| (The process followed was similar to that provided in Example 1.) | |

### EXAMPLE 14:

Product from Example 13 was added to yogurt. Particles formed a fluffy soft gel. This is an example of a calcium alginate hydrogel composition.

### EXAMPLE 15:

The following formulation was processed via spray drying:

| | % by weight | |
|---|---|---|
| | Formula A | Formula B |
| Water | 62 | 62 |
| Strawberry Flavor | 11.25 | 11.25 |
| Modified Starch | 21.5 | 21 |
| Gelatin | 5.25 | 5.25 |
| Transglutaminase | 0 | 0.5 |

### EXAMPLE 16:

Products from Example 15 were suspended in water and examined under the microscope. The majority of particles from Formula A dissolved although a few gel-like clusters were formed in sections where there was a collection of powder. Particles from Formula B swelled and formed stable hydrogels.

The description provided above is not limiting to the scope of the invention. Many other permutations may be specified once the basic concept is understood. These are included although they are not outlined in detail.

### EXAMPLE 17:

The following formulation was processed via extrusion, cooled, and sized:

| | % by weight |
|---|---|
| Strawberry Flavor | 10 |
| Silicon Dioxide | 5 |
| Emulsifier | 2 |
| Sorbitol | 30.8 |
| Gelatin | 50 |
| Transglutaminase | 2 |
| Color lake red #40 | 0.2 |

The extrusion barrel zone temperatures ºC were: 40-50, 50-60, 55-65, 60-75, 90-105. The final particles were irregular in shape and their size was -8/ + 20 mesh.

### EXAMPLE 18:

Product from Example 17 was placed in the bottom of a beaker. Beer at refrigerated temperature (Budweiser purchased at a local store) was added. The particles began sinking and floating after about 30 seconds, and continued this active motion for at least 4 minutes.

### EXAMPLE 19:

Product from Example 17 was placed in the bottom of a beaker. Seltzer at refrigerated temperature (purchased at a local store) was added. The particles began sinking and floating after about 30 seconds, and continued this active motion for at least 4 minutes.

### EXAMPLE 20:

Product from Example 17 was placed into a cold bottle of beer (Budweiser purchased at a local store). The bottle was re-capped and stored in the refrigerator for approximately 15 minutes. At this time, all of the particles were at the bottom of the bottle. When the bottle was uncapped, the particles immediately began active motion, and continued this until the bottle was re-capped.

### EXAMPLE 21:

The following formulation was processed via extrusion:

| | % by weight |
|---|---|
| Peppermint Flavor | 10 |
| Silicon Dioxide | 5 |
| Emulsifier | 2 |
| Sorbitol | 30.8 |
| Gluten | 50 |
| Hydrolyzed gluten | 0 |
| Transglutaminase | 2 |
| Color lake blue #1 | 0.2 |

| | |
|---|---|
| (The process followed was similar to that provided in Example 1.) | |

### EXAMPLE 22:

Commercial apple sauce was heated in a water bath to approximately 80ºC. Product from Example 21 was added (2% by weight of the apple sauce) and stirred to obtain a uniform distribution. Mixture was kept heated in the water bath for at least 40 minutes. The mixture was stirred vigorously. The hydrogel particles were observed to retain color and particle integrity.

### EXAMPLE 23:

The following formulation was processed via extrusion:

| | % by weight |
|---|---|
| Peppermint Flavor | 10 |
| Silicon Dioxide | 5 |
| Emulsifier | 2 |
| Sorbitol | 30.8 |
| Gluten | 25-35 |
| Hydrolyzed gluten | 15-25 |
| Transglutaminase | 2 |
| Color lake blue #1 | 0.2 |

| | |
|---|---|
| (The process followed was similar to that provided in Example 1.) | |

### EXAMPLE 24:

Quick cook oats was mixed with the product of Example 23 (2% by weight of the dry oats). The mixture is added to boiling hot water and heat reduced to medium and cooked for an additional 2-4 minutes with occasional stirring. The ratio of the oat mixture to water is about 1:2 by volume. At the end of 4 minutes, the hydrogel particles were observed to retain color and particle integrity.

## Claims

1. A method for preparation of a hydrogel comprising
providing a functional polymer;
providing an active ingredient selected from the group consisting of therapeutic agents, diagnostic agents and flavors;
admixing said functional polymer and said active ingredient;
extruding the mixture of the functional polymer and the active ingredient;
the preceding steps being performed in dry conditions;
following by forming a hydrogel in-situ by contacting the extruded material with water.

2. A method for preparation of a hydrogel as in claim 1, wherein the functional polymer is selected from the group consisting of alginate, gelatin, gluten, starch, agar, xanthan gum, gellan gum, pectin, guar gum, hydroxypropyl methylcellulose (HPMC), methyl cellulose, microcrystalline cellulose, soy protein, whey protein, casein, collagen and hydrolyzed gelatin.

3. A method for preparation of a hydrogel as in claim 1 or claim 2, wherein the amount of functional polymer used is from about 1 to about 60 percent by weight prior to the uptake of water.

4. A method for preparation of a hydrogel as in claim 1 or claim 2, wherein the amount of functional polymer used is from about 7 to about 50 percent by weight prior to the uptake of water.

5. A method for preparation of a hydrogel as in any preceding claim, wherein the active ingredient is a hydrophobic material.

6. A method for preparation of a hydrogel as in any preceding claim, wherein the amount of active ingredient used is from about 5 to about 40 percent by weight prior to the uptake of water.

7. A method for preparation of a hydrogel as in any of claims 1 to 5, wherein the amount of active ingredient used is from about 10 to about 20 percent by weight prior to the uptake of water.

8. A method for preparation of a hydrogel as in any preceding claim, wherein glutaraldehyde, formaldehyde are cross-linking agents.

9. A method for preparation of a hydrogel as in any preceding claim, further comprising a functional additive.

10. A method for preparation of a hydrogel as in claim 9, wherein the amount of functional additive used is from about 0.1 to about 20 percent by weight prior to the uptake of water.

11. A method for preparation of a hydrogel as in claim 9, wherein the amount of functional additive used is from about 0.05 to about 10 percent by weight prior to the uptake of water.

12. A method for preparation of a hydrogel as in claim 8 or any of claims 9 to 11, wherein the functional additive is calcium gluconate.

13. A method for preparation of a hydrogel as in claim 12, wherein calcium gluconate is a cross-linking agent.

14. A method for preparation of a hydrogel as in any preceding claim, further comprising a colorant.

15. A method for preparation of a hydrogel as in claim 14, wherein the amount of colorant used is from about 0.01 to about 10 percent by weight prior to the uptake of water.

16. A method for preparation of a hydrogel as in claim 14, wherein the amount of colorant used is from about 0.1 to about 5 percent by weight prior to the uptake of water.

17. A method for preparation of a hydrogel as in any of claims 14 to 16, wherein said hydrogel composition retains color in high moisture systems.

18. A method for preparation of a hydrogel as in any of claims 14 to 17, wherein said hydrogel composition retains color when heated at a temperature of from about 75°C to about 95°C.

19. A method for preparation of a hydrogel as in any of claims 14 to 18, wherein the colorant is a botanical material selected from the group consisting of ground tea leaves, dried parsley, dried red bell pepper, fruit powders, annatto, turmeric and beta-carotene.

20. A method for preparation of a hydrogel as in any preceding claim, further comprising an emulsifier.

21. A method for preparation of a hydrogel as in any preceding claim, further comprising providing transglutaminase as a cross-linking agent in dry conditions.

22. A method for preparation of a hydrogel as in claim 21, wherein the concentration of transglutaminase is less then about 200 units.

23. A method for preparation of a hydrogel as in any preceding claim, further comprising providing a heating source to melt the mixture, creating the desired shape and size of the melted particles, cooling the melted particles, the preceding steps being performed in dry conditions and forming a hydrogel in-situ by contacting the melted particles with water.

24. The product prepared by the method of any preceding claim.

25. A method for preparation of a hydrogel as in any preceding claim, wherein the active ingredient is a fragrance.

26. A method for preparation of a hydrogel as in any preceding claim, wherein the polymer has a molecular weight of less than 250,000.

27. A method for preparation of a hydrogel as in any preceding claim, wherein the polymer has a molecular weight of less than 40,000.

28. A method for preparation of a hydrogel as in any preceding claim, wherein radiation energy is not used to achieve cross-linking.

29. A method for delivering an active ingredient to an ingestible composition comprising preparing a hydrogel as in any preceding claim and providing said hydrogel into said ingestible composition.

30. Hydrogel particles comprising a functional polymer, a functional additive and an active ingredient, whereby the hydrogel particles, when incorporated into a carbonated beverage, exhibit movement within the beverage due to the release of carbonation in the beverage.

31. The hydrogel particles of claim 30 wherein the functional polymer selected from the group consisting of alginate, gelatin, gluten, starch, agar, xanthan gum, gellan gum, pectin, guar gum, hydroxypropyl methylcellulose (HPMC), methyl cellulose, microcrystalline cellulose, soy protein, whey protein, casein, collagen and hydrolyzed gelatin.

32. The hydrogel particles of claim 30 or claim 31 wherein the active ingredient is selected from the group consisting of extracts, caffeine, nutritional supplements and flavors, regular and high-intensity sweeteners, coolers and other taste-modifying compounds and acids.

33. The hydrogel particles of any of claims 30 to 32, wherein the functional additive is cross-linking agent selected from the group consisting of glutaldehyde, formaldehyde calcium gluconate or enzymes.

34. The hydrogel particles of claim 33 wherein the cross-linking agent is transglutaminase.

35. The hydrogel particles of any of claims 30 to 34 further comprising a colorant.

36. The hydrogel particles of any of claims 30 to 35 further comprising titanium dioxide.

37. The hydrogel particles of any of claims 30 to 36, wherein said hydrogel composition retains color in carbonated beverage systems.

38. The hydrogel particles of any of claims 30 to 37 further comprising an emulsifier.

39. A dry beverage mix comprising dry beverage mixture and hydrogel particles wherein the hydrogel particles comprise a functional polymer, a functional additive and an active ingredient; whereby the hydrogel particles, when incorporated into a carbonated beverage, exhibit movement within the beverage due to the release of carbonation in the beverage.

40. A method for delivering an active ingredient to a carbonated beverage comprising preparing hydrogel in the following manner:
providing a functional polymer;
providing an active ingredient selected from the group consisting of extracts, caffeine, nutritional supplements, and flavors;
admixing said functional polymer and said active ingredient;
extruding the mixture of the functional polymer and the active ingredient;
the preceding steps being performed in dry conditions;
followed by forming a hydrogel in-situ by contacting the extruding material with water; and providing the hydrogel into the carbonated beverage.
